# EUROPEAN PATENT APPLICATION

(11) **EP 4 183 380 A1**
(43) Date of publication of application: **24.05.2023**
(21) Application number: 21209553.3
(22) Date of filing: 22.11.2021
(51) Int. Cl.: A61F 11/00, B33Y 10/00, B33Y 80/00

(54) **PATIENT-SPECIFIC, DRUG RELEASING, COMPRESSIBLE IMPLANT WITH IN-TEGRATED HANDLE FOR INSERTION INTO A BODY CAVITY AND METHOD FOR ITS MANUFACTURE**

(71) Applicant: Medizinische Hochschule Hannover, 30625 Hannover (DE); HörSys GmbH, 30625 Hannover (DE)
(72) Inventor: SCHEPER, Verena, 31555 Suthfeld (DE); DOLL, Theodor, 30916 Isernhagen (DE); MATIN, Farnaz, 30175 Hannover (DE); John, Samuel, 30559 Hannover (DE); LENARZ, Thomas, 30559 Hannover (DE)
(74) Representative: Kröncke, Rolf

(57) **Abstract**

The invention relates to a patient-specific implant for insertion into a body cavity of a patient, for example into the round window niche of the inner ear. The invention also relates to a method of manufacturing such an implant.

## Description

The invention relates to a patient-specific implant for insertion into a body cavity of a patient, for example into the round window niche of the inner ear. The invention also relates to a method of manufacturing such an implant.

There are already proposals for the manufacture of patient-specific implants. However, the suitability of such known implants for insertion into a patient's body cavity is severely limited because either
a) their shape is not based on the individual anatomic shape, or
b) the correct orientation and positioning is difficult due to missing indicators, or
c) the body cavity is accessible only through a narrowed body opening, such is the case for the round window niche.

It is an object of the invention to provide an improved implant to address (a), (b), or (c), which can be inserted simply and correctly orientated and remain reliably at the target position due to the patient individual anatomical shape, even in body cavities which are accessible only via a narrowed body opening. Furthermore, an advantageous method for manufacturing such an implant is to be disclosed.

This object is achieved by a patient-specific implant for insertion into a body cavity of a patient, the implant having an implant body and a handle attached directly or indirectly to the implant body, wherein the handle is adapted for holding the implant during insertion into the body cavity, the implant body having an outer contour which corresponds predominantly to the inner contour of the body cavity of the patient, wherein at least the implant body contains a medical active substance which is deliverable from the implant body to the patient. Such an implant can be placed reliably and safely in the body cavity. The implant can be held by the handle and guided by the user or a robot during the insertion process. The handle can also support the user in guiding the implant during insertion into the body cavity. At the implantation site, the implant body can then deliver the medical active substance to the patient.

The implant is a patient-specific implant which is formed in a shape adapted to the anatomical shape of the body cavity of a patient where the implant shall be placed.

According to an advantageous embodiment of the invention, the implant body is formed as a solid body having substantially no air pockets. This has the advantage that the implant body can be provided both mechanically stable and to a certain extent compressible for the insertion process.

According to an advantageous embodiment of the invention, the implant body has the same or a higher compressibility than the handle. This has the advantage that the implant can be reliably held and guided via the more rigid handle compared to the implant body, while the implant body can also be guided through constricted areas to the body cavity due to its higher compressibility. For example, the implant body may be provided from a material having a higher compression modulus than the material of the handle.

According to an advantageous embodiment of the invention, the implant body is elastically compressible by at least 20%. In this way, the implant body can have sufficient softness when inserted into the body cavity. For example, the implant body can be compressed to at least 20% less volume than in the uncompressed state without permanent damage.

According to an advantageous embodiment of the invention, the implant body has the same or a greater elasticity than the handle. This allows the implant body to conform well to the body cavity and, during the insertion process, to the possibly narrower body caves upstream of the body cavity. For example, the implant body may be made of a material with a greater modulus of elasticity than the material of the handle.

According to an advantageous embodiment of the invention, the implant handle may be wider than the implant body in an extent predominantly orthogonal to the insertion direction in order to function as a depth stop for avoiding too deep over-insertion of the whole implant. In this case the implant handle may remain outside of the body cavity resting on the constriction at the entry of the body cavity to make an over-insertion impossible.

According to an advantageous embodiment of the invention, the implant handle may be individually shaped such that it rests in a form-fit at on the constriction at the entry of the body cavity while the implant body has reached the correct implantation depth and the correct position in the body cavity.

According to an advantageous embodiment of the invention, the implant has a marking by which the spatial position and/or orientation of the implant during the implanting process in the body cavity is recognizable to the user. This has the advantage that the position and/or orientation of the implant in the 3D space can be checked by the user at any time during the implantation process and the correct implantation direction can be ensured. Such a marking thus serves as an orientation aid for the user when implanting the implant into the body cavity. The marking may, for example, be in the form of an arrow. It is advantageous if the marking is arranged on the handle, because it is then clearly visible to the user during the entire implantation process. The shape of the handle itself can fulfill the role of a marking regarding position and/or orientation of the implant.

According to an advantageous embodiment of the invention, the handle is formed as a position and/or orientation aid which supports the user in guiding the implant during insertion into the body cavity. For example, the handle itself can be the aforementioned marker.

According to an advantageous embodiment of the invention, the implant body and/or the handle may be biodegradable. Accordingly, the implant body and/or the handle may slowly decompose in the body cavity, which has the advantage that no additional intervention is required for explantation of the implant body and/or the handle. The entire implant may also be biodegradable.

According to an advantageous embodiment of the invention, the implant body has a multilayer structure of medical active substance-loaded material with medical active substance concentrations and/or medical active substances differing in the layers. In this way, a time-programmed medical active substance release can be realized optionally with respect to the medical active ingredient concentration and/or with respect to the type of medical active ingredient.

According to an advantageous embodiment of the invention, active substance-producing cells are integrated in the implant body, by means of which the medical active substance that can be delivered to the patient can be produced. In this way, a constant delivery of the active substance to the patient can be ensured over a longer period of time.

According to an advantageous embodiment of the invention, at least the implant body is manufactured by means of an additive manufacturing process. This allows a particularly precise patient-specific provision of the implant body or the implant. The entire implant may also be manufactured using an additive manufacturing process. The additive manufacturing process can be, for example, a 3D printing process, e.g. laser printing, extrusion printing, inkjet printing.

The object of the invention is further achieved by a method for manufacturing a patient-specific implant of the type explained above, comprising the following steps:
a) Reading patient data into a computer program, the patient data describing the shape of the body cavity into which the implant is to be placed,
b) Modeling of the implant body in the computer program according to the shape of the body cavity and optionally the shape of the constriction at the entry of the body cavity using the patient data,
c) Adding a handle to the implant body in the computer program and optionally modeling the shape of the handle using the patient data to match shape of the constriction at the entry of the body cavity
d) Outputting the implant modelled in the computer program to an additive manufacturing device.

The previously explained advantages can also be realized by this method. The patient data can be obtained, for example, by means of an examination process of the patient performed before the execution of the method, e.g. by a radiological three-dimensional examination, in particular by an examination by means of computer tomography, magnetic resonance tomography and/or digital volume tomography.

The invention is explained in more detail below with reference to exemplary embodiments using drawings.

The drawings show in
Figure 1 a schematic representation of part of the inner and middle ear of a patient,
Figure 2 a patient-specific implant,
Figure 3 a marking element,
Figure 4 a manufacturing process of the implant.

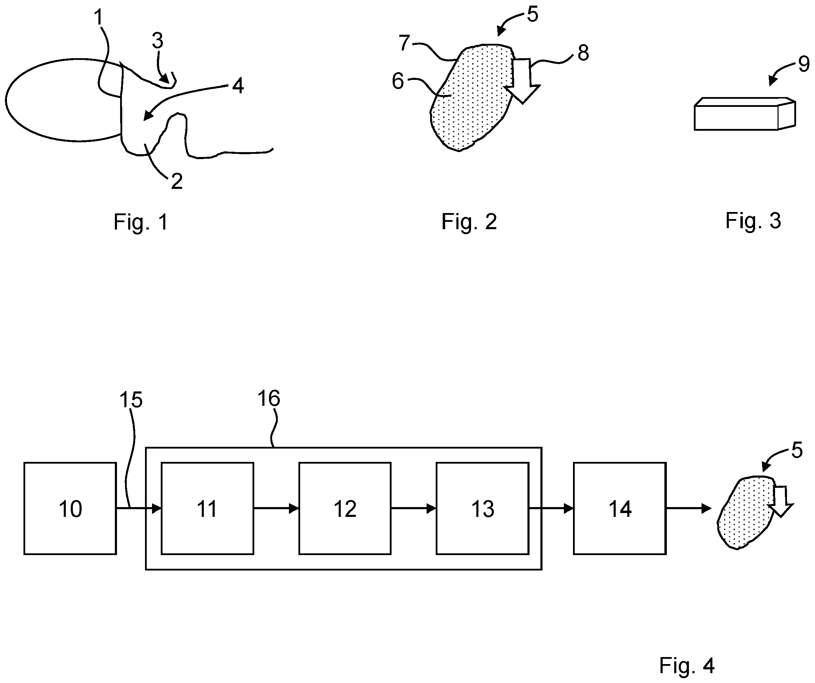

Figure 1 shows a schematic representation of an area of the inner and middle ear of a living organism, e.g. a human or an animal. The round window niche 2 can be seen, which is separated from other areas of the inner ear, in particular the Scala tympani, by the round window membrane 1 which can be sometimes a pseudomembrane. If an implant is to be placed in the round window niche 2, it must first be passed through the isthmus 3 of the round window niche 2 according to the arrow 4, i.e. a constricted area, before it can be placed in the larger round window niche 2. For this purpose, it is advantageous if the implant or at least the implant body has sufficient compressibility so that it can be guided through the isthmus 3 without damaging the patient. After passing the isthmus 3, the implant body expands again and fits into the round window niche 2.

Figure 2 shows a patient-specific implant 5 designed for insertion into the round window niche 2. The patient-specific implant 5 has an implant body 7 and a handle 8 attached to the implant body 7. The handle 8 can be used for holding and guiding the implant body 7 during the insertion process. The handle 8 may have a marking as an orientation aid for the user or may be designed in the form of such a marking, for example in the form of an arrow. At least one medical active substance 6 is present in the implant body 7, which can be delivered to the patient by the implant body 7 arranged in the round window niche 2, for example by diffusing the medical active substance 6 through the round window membrane 1 into the Scala tympani of the inner ear.

Figure 3 shows an advantageous design of a marking element 9 which can be arranged on the implant 5 as an orientation aid, for example on the handle 8. The marking element 9 can also form the handle 8 at the same time. The shape of the marking element 9 can be designed as a cuboid, with one side being pointed, in order to obtain information about the orientation of the implant.

Figure 4 shows the manufacturing process of the implant 5 by computer aided manufacturing. Prior to the actual manufacturing process, patient data are determined in a step 10, e.g. by means of an X-ray three-dimensional examination. The patient data 15 are read into a computer program 16. Within this computer program 16, various steps are performed. In a step 11, a modeling of the implant body 7 is performed, for example by selecting and segmenting in the patient data 15 the relevant region of the body cavity into which the implant 5 is to be inserted. In a subsequent step 12, a handle for handling by the surgeon is added to the implant body modelled in the computer program. Based on this, in a step 13, the corresponding print output file for an additive manufacturing device can be generated, for example an STL file. This print file is then output from the computer program 16 to an additive manufacturing device 14, by which the real implant 5 is then produced by means of additive manufacturing.

The invention describes a new class of implants that can be inserted as a solid body into the round window niche (RWN) and can locally deliver active substances there for the treatment of various inner ear diseases. For atraumatic placement, secure retention and effective drug delivery, it is advantageous to customize the implants to the geometry of the individual round window niche. In order to be able to be inserted into the cavity, it is advantageous if the implant is compressible and has a marking for the correct implantation direction.

The implant according to the invention may thus having one, more or all of the following features:
- Full body
- Individual shape and size specific to the patient
- at the same time mechanically stable and compressible for insertion
- medical active substance containing and releasing
- Marking for orientation
- Grab handle

To avoid explantation of the implants, they can be made of biodegradable materials. Targeted drug release into the inner ear may also be implemented. Additionally, the implant according to the invention may have one, more or all of the following features:
1) degradable
2) induced dissolution or acceleration of degradation.
3) Target tissue-oriented medical active substance release
4) time-programmed release of the medical active substance by means of a layer-by-layer structure of active ingredient-laden material with layer-by-layer different active ingredient concentrations or different active ingredients.
5) Integration of drug-producing cells

Round window niche implants allow the treatment of the inner ear with active substances that diffuse from the implant through the round window membrane into the inner ear. Thus, pathologies such as Menier's disease, hearing loss and cochlear implant-associated trauma reactions can be treated. To date, it is not possible to diffuse drugs into the inner ear in a controlled manner, since the drug-releasing implant surface is not adapted to the round window membrane of the individual patient. There are considerable variations in size and shape, as well as a pseudomembrane in some cases. The implant of the invention is the first drug-releasing system that is adapted to the individual anatomy of the patient and can therefore be safely implanted in the round window niche to deliver its active ingredients to the inner ear in an effective and controlled manner. Its compressibility allows atraumatic insertion through the isthmus into the round window niche, where it remains, as the expansion after insertion prevents it from slipping out.

The handle may have an arrow shape and thus also serves to orient the surgeon in which direction the implant is to be placed. The shape and thickness of the handle can be varied so that an optimal support structure for the individualized implant body can be printed.

In a manufacturing step, the handle for the implant is created on the surface of the implant facing the middle ear (step 12 in Figure 4). The shape of the handle can be chosen as a cuboid with one side pointed to provide information about the orientation of the implant. By default, this side points in the direction of the basal rotation of the cochlea at the round window to allow the surgeon to implement the planned fit in the niche. The orientation and dimensions of the handle can be adjusted by the user, while the software ensures that there is no overlap with bony structures of the middle ear, based on the specified threshold. The dimensions of the handle can be selected to be small enough that, if necessary, only the arrow mark is imaged and no more handle is required. Accordingly, the implant can subsequently be printed with or without a handle, but with has a guide for the surgeon.

In general, biopolymers, synthetic polymers, polymer blends, nanocomposites, functional polymers and cell-loaded systems can be used as materials for the implant body and/or the handle. The materials to be printed should be dimensionally stable on the one hand, but on the other hand compressible in order to be introduced into the cavity. Hydrogels are suitable for this purpose, as they have a lattice structure in the linked state, which enables the inclusion of active substances with subsequent diffusion out of the material. At the same time, the material should be degradable in order to avoid explantation of the implant after successful therapy.

Suitable materials are for example
- Silicone
- Alginate
- Cellulose
- Chitosan
- Polyethylene glycol (PEG)
and their combinations with each other as well as with other non-flexible materials such as
Poly(lactic acid) (PLA), Poly(glycolic acid) (PGA), Poly(lactic-co-glycolic acid) (PLGA), Poly(caprolactone) (PCL), Poly(amide, Poly(anhydride), Poly(phosphazene), Poly(dioxanone)

The materials are either mixed with medical active substances prior to manufacture or the implant is filled with medical active substances by diffusion after manufacture.

The amount of medical active substances to be introduced depends on the pathology addressed and varies accordingly.

Some possible medical active substances are listed in Table 1 below:

**Table 1: Exemplary drug classes and active ingredients.**

| **Anti-inflammatory** | **Immunosuppressants** |
|---|---|
| Dexamethasone, dexamathasone sodium phosphate | Mitomycn |
| Triamcinolone, Methylprednisolone | Sirolimus |
| Mometasone Fuorate | |
| Fluticasone proprionate, Etanercept, Diclofenac | |

| **Antioxidant** | **Antibacterial** |
|---|---|
| Butylhydroxyltoluene, Edaravon | Ciprofloxacin |

| **H3 antihistamine** | **Neuroprotective** |
|---|---|
| Betahistine | BDNF, GDNF, NT-3, IGF, NTF, FGF and agonists of the corresponding receptors |

The implant can be manufactured, for example, by means of additive manufacturing processes listed below.
3.3.1 Laser printing:
   - Stereolithography (SLA)
   - two-photon polymerization (2PP)
   - laser-induced forward transfer
3.3.2 Extrusion printing
   - Fused deposition modeling (FDM)
   - direct ink writing
3.3.3 inkjet printing
   - Selective laser sintering (SLS)
   - Digital light processing (DLP) technology

The implants should be as biodegradable as possible = bioresorbable = bioabsorbable. They should either dissolve independently in the body or be induced to dissolve.

Reduced dissolution of alginate can be done by injection of alginate lyase into the middle ear. Chitosan can be dissolved by injection of a low pH solution. It must be noted that the solutions must be biocompatible.

Immortalized, drug-producing cells can be introduced into the implant. The factors to be produced include, but are not limited to, growth factors and other proteins, as well as antioxidant molecules, which can be neuroprotective as well as antioxidant and thus protect specific inner ear structures from degeneration. The cells should survive in the implant for at least 4 weeks. The drugs must diffuse from the implant into the inner ear, but the cells must be firmly entrapped in the material to avoid an immune response from the patient. Cells of any origin such as stem cells, fibroblasts or human umbilical vein endothelial cells (HUVEC) can be introduced.

## Claims

1. A patient-specific implant (5) for insertion into a body cavity (2) of a patient, the implant (5) having an implant body (7) and a handle (8) attached directly or indirectly to the implant body (7), wherein the handle (8) is adapted for holding the implant (5) during insertion into the body cavity (2), the implant body (7) having an outer contour which corresponds predominantly to the inner contour of the body cavity (2) of the patient, **characterized in that** at least the implant body (7) contains a medical active substance (6) which is deliverable from the implant body (7) to the patient.

2. Implant according to claim 1, **characterized in that** the implant body (7) is formed as a solid body having substantially no air pockets.

3. An implant according to any one of the preceding claims, **characterized in that** the implant body (7) has the same or a higher compressibility than the handle (8).

4. An implant according to any one of the preceding claims, **characterized in that** the implant body (7) is elastically compressible by at least 20%.

5. Implant according to any one of the preceding claims, **characterized in that** the implant body (7) has the same or a greater elasticity than the handle (8).

6. Implant according to any one of the preceding claims, **characterized in that** the implant handle (8) may be wider than the implant body (7) in an extent predominantly orthogonal to the insertion direction in order to function as a depth stop for avoiding too deep over-insertion of the whole implant (5).

7. Implant according to any one of the preceding claims, **characterized in that** the implant handle (8) may be individually shaped such that it rests in a form-fit at on the constriction at the entry of the body cavity (2) while the implant body (7) has reached the correct implantation depth and the correct position in the body cavity (2).

8. Implant according to any one of the preceding claims, **characterized in that** the implant (5) has a marking (9) by which the spatial position and/or orientation of the implant (5) during the implanting process in the body cavity (2) is recognizable to the user.

9. Implant according to claim 8, **characterized in that** the marking (9) is arranged on the handle (8).

10. Implant according to any one of the preceding claims, **characterized in that** the handle (8) is formed as a position and/or orientation aid which supports the user in guiding the implant (5) during insertion into the body cavity (2).

11. Implant according to any one of the preceding claims, **characterized in that** the implant body (7) and/or the handle (8) is biodegradable.

12. Implant according to any one of the preceding claims, **characterized in that** the implant body (7) has a multilayer structure of medical active substance-loaded material with medical active substance concentrations and/or medical active substances differing in the layers.

13. Implant according to any one of the preceding claims, **characterized in that** active substance-producing cells are integrated in the implant body (7), by means of which the medical active substance (6) that can be delivered to the patient can be produced.

14. Implant according to any one of the preceding claims, **characterized in that** the implant (5) is adapted for insertion into the round window niche (2) of the inner ear of a patient.

15. Implant according to any one of the preceding claims, **characterized in that** at least the implant body (7) is manufactured by means of an additive manufacturing process.

16. A method of manufacturing a patient-specific implant (5) according to any one of the preceding claims, comprising the steps of:
a) Reading patient data (15) into a computer program (16), the patient data (15) describing the shape of the body cavity (2) into which the implant (5) is to be placed,
b) Modeling of the implant body (7) in the computer program (16) according to the shape of the body cavity (2) and optionally the shape of the constriction at the entry of the body cavity (2) using the patient data (15),
c) Adding a handle (8) to the implant body (7) in the computer program (16) and optionally modeling the shape of the handle (8) using the patient data (15) to match shape of the constriction at the entry of the body cavity (2) in the computer program (16),
d) Outputting the implant modelled in the computer program (16) to an additive manufacturing device (14).
